# EUROPEAN PATENT APPLICATION

(11) **EP 0 870 463 A2**
(43) Date of publication of application: **14.10.1998**
(21) Application number: 98106652.5
(22) Date of filing: 10.04.1998
(51) Int. Cl.: A61B 1/00, A61B 5/055, G01R 33/28

(54) **Device having low magnetic susceptibility**

(30) Priority: 11.04.1997 US 43647 P; 11.04.1997 US 43650 P
(71) Applicant: WILSON GREATBATCH LTD., Clarence New York 14031 (US)
(72) Inventor: Allred, III, Jimmie B., Skaneateles, New York 13152 (US); Mott, Richard W., East Amherst, New York 14051 (US); Mozeko, Charles L., Tonawanda, New York 14150 (US)
(74) Representative: Koepe, Gerd L., Dipl.-Chem.

(57) **Abstract**

A device constructed of materials including metal components having very low magnetic susceptibilities is described. The device is particularly useful in the vicinity of the strong magnetic field of an MRI scanner.

## Description

### CROSS-REFERENCE TO RELATED APPLICATION

The present application is based on provisional application Serial Nos. 60/043,647 and 60/043,650, both filed April 11, 1997.

### BACKGROUND OF THE INVENTION

### 1. Field of the Invention

The present invention generally relates to a hand held medical device. More particularly, the present invention relates to an endoscope, and still more particularly to an endoscope that is constructed of materials having relatively low magnetic susceptibilities. This provides the endoscope of the present invention as a useful instrument in the vicinity of a magnetic resonance scanner.

### 2. Prior Art

The prior art is replete with various types of hand held medical devices, such as flashlights, made of metal materials that are not useful in the presence of the strong magnetic fields of a magnetic resonance scanner. Examples include U.S. Patent Nos. 1,067,646 to Downey; 1,877,077 to Stevens; 2,459,702 to Hipwell et al.; 2,651,763 to Grimsley; 3,890,498 to Toth, Sr.; 4,203,150 to Shamlian; 4,237,527 to Breedlove; 4,286,311 to Maglica; 5,593,222 to Maglica; and 5,601,359 to Sharrah et al.

U.S. Patent No. 4,607,623 to Bauman describes a hand held laryngoscope constructed of non-ferrous materials such as ABS with the electrically conductive portions provided by first applying a thin copper layer to the ABS followed by electroless plating and then electrolytically plating another copper layer to form a conductive layer about 0.5 to 2 mils thick. A thin layer of aluminum is subsequentially applied to the copper coating in those areas intended to be reflective. The batteries powering this device are not further described, but may be of a nickel/cadmium type commonly used for such applications. Nickel/cadmium batteries are not considered to be relatively nonmagnetic.

There is therefore, needed an endoscope that is predominantly constructed of metal so that the instrument is capable of withstanding the abusive treatment conditions which surgical instruments are sometimes subjected to. For this purpose, the endoscope of the present invention is constructed largely of metal components. However, with ever increasing use of magnetic resonance scanning to aid medical personnel during pre- and post- clinical and surgical procedures, the metal components must be constructed of materials that have as low a magnetic susceptibility as possible.

### SUMMARY OF THE INVENTION

For a surgical instrument to be suitable for use in an MRI environment, the instrument must be constructed of materials that are compatible with body fluids, compatible with solutions used in surgery, e.g., saline, betadine and others, exhibit stability and durability during autoclave, have sufficient material strength to allow for successful surgery, exhibit toughness to sustain loaded conditions, e.g., puncture of the skin or unintended impacts, provide a magnetic signature sufficient to generate an artifact of the same size and shape as the instrument, provide a magnetic signature such that image distortion is prevented and provide a magnetic signature such that deflection of the instrument in the presence of the MRI magnet is precluded. For that reason, brass is a useful material for the present device because it has a low magnetic susceptibility as well as meeting the other stated requirements. However, brass is not corrosion resistant so those parts made of brass are typically plated with a corrosion resistant material such as chrome. Chrome is very impact resistant and has a low magnetic susceptibility. Typically, chrome is plated on brass using a nickel strike. However, nickel has a magnetic susceptibility about 600 and is, therefore, unacceptable for use in the present invention. Unexpectably, the brass material of the present invention is able to be chrome plated without first providing a nickel coating on the brass.

Other coating materials useful with the present invention include titanium nitride and parylene. Titanium nitride is a hard ceramic coating with toughness characteristics similar to chrome and that is typically physical vapor deposited. Parylene is a physical vapor deposited polymeric coating that imparts corrosion resistance and lubricity, if required. However, it is not quiet as tough or impact resistant as chrome and titanium nitride.

The endoscope of the present invention is constructed of materials including metal components such as brass and beryllium copper having very low magnetic susceptibilities. Those parts not made of metal are preferably formed of a non-magnetic thermoplastic material, for example an acetal compound such as DELRIN.

These and other aspects of the present invention will become more apparent to those skilled in the art by reference to the following description and to the appended drawings.

### BRIEF DESCRIPTION OF THE DRAWINGS

Fig. 1 is a perspective view of an endoscope 10 according to the present invention.

Fig. 2A is a cross-sectional view of a housing 12 of the endoscope 10 shown in Fig. 1 supporting a lens/light conduit 14, a light guide tower 16 and an eye piece 18.

Fig. 2B is a cross-sectional view of an intermediate section of the lens/light conduit 14 of the endoscope shown in Fig. 1.

Fig. 2C is a cross-sectional view of a distal end of the lens/light conduit 14.

Fig. 3 is an end view of the distal end of the endoscope 10.

### DETAILED DESCRIPTION OF THE INVENTION

As defined in this application, the word "distal" is used to describe that portion of the endoscope that extends away from the user holding the handle, and the word "proximal" is used to describe that portion of the endoscope that extends toward the user holding the device by the handle.

Further, as defined in this application, the term metal defines any of various opaque, fusible, ductile, and typically lustrous substances that are good conductors of electricity and heat, form cations by loss of electrons, and yield basic oxides and hydroxides. A metallic compound is a material of, relating to, or being a metal.

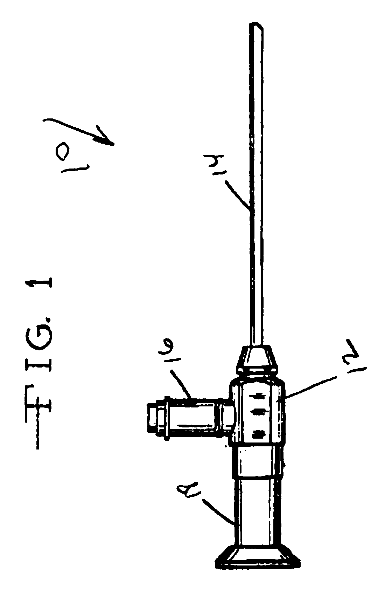

Turning now to the drawings, Figs. 1 to 3 show an endoscope 10 having low magnetic susceptibility characteristics according to the present invention. It should be understood that endoscope 10 is exemplary of many types of endoscopes according to the present invention that are useful for pre- and post-clinical applications, especially in an environment proximate the strong magnetic field emitted by a magnetic resonance imaging (MRI) scanner. Such endoscopes include, but are not limited to, sinuscopes, arthroscopes, laparascopes, laryngoscopes and hysteroscopes. However, in a broader sense, endoscope 10 is exemplary of any hand held medical device that is intended for use in the proximity or under the influence of the strong magnetic field emitted by a MRI scanner. In that respect, in addition to describing a hand held medical device, the present invention is directed to a material, and particularly an alloy of copper and zinc as a brass material, from which such devices are constructed that allows them to be used in the vicinity of an MRI scanner.

Endoscope 10 is comprised of a housing 12 that serves as a handle for the device and supports a lens/light conduit 14, a light guide tower 16 and an eye piece 18. The housing 12, the lens/light tube 14 and the light guide tower 16 are constructed of brass, and particularly a brass consisting essentially of, by weight percent, 62 to 65% copper, <0.02% cadmium, <0.03% iron, <0.03% tin, remainder zinc. This brass material is specifically formulated as a material that is acceptable for fabrication of a hand held, interventional medical device used in proximity to the magnetic field emitted by a MRI scanner. Further those brass parts which form the external parts of the medial device are chrome plated.

The housing 12 has opposed end walls 20 and 22 meeting an intermediate annular side wall 24. End wall 20 threadenly engages with a frusto-conically shaped nut 26 having an opening sized to mount the lens/light conduit 14 of the housing. The opposite end wall 22 receives a bushing 28 in a tightly fit relationship. A sleeve 30 extends rearwardly from the end wall 22.

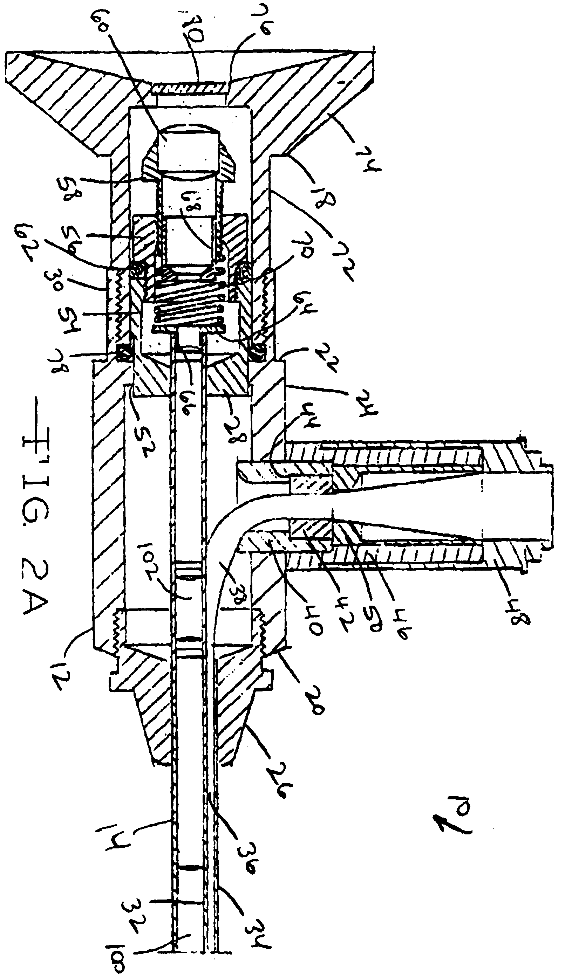

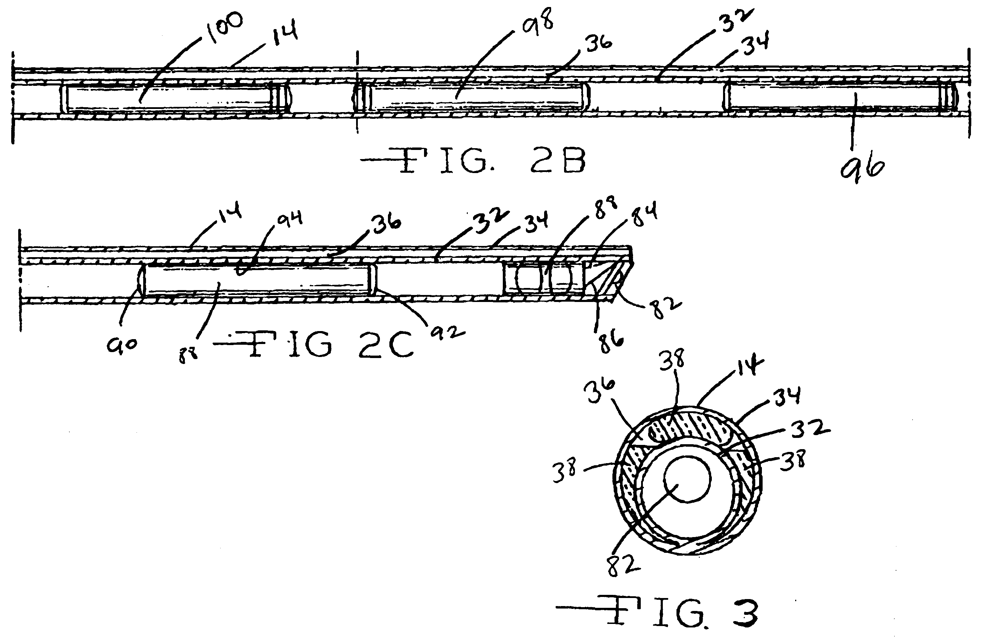

As shown in Figs. 2A to 2C, lens/light conduit 14 comprise a lens tube 32 disposed off centered and inside of a light tube 34. The lens tube 32 is somewhat longer than the light tube 34, which terminates inside the nut 26. An annulus 36 is provided between approximately 350° about the outside of the lens tube 32 and the inside of the light tube 34. The annulus 36 provides a channel for a plurality of light conducting fiber optic cables 38 that run from the distal end of the light tube 34 to the terminus thereof and into the light guide tower 16.

The light guide tower 16 comprises a first, inner sleeve 40 having an annular polymeric gasket 42 seated on a ledge 44 and surrounding the light cables 38 in a closely spaced relationship. An outer light cable tube 46 contacting the side wall 24 of the housing 12 surrounds the inner sleeve 40 and extends to an adapter 48 that serves as a quick-disconnect for connecting the endoscope 10 to a light source (not shown). An insert 50 is provided inside the outer light cable tube 46 and serves to maintain the ends of the light cables 38 in proper alignment with the light source.

The bushing 28, which is received in an opening 52 in the end wall 22 of the housing 12, has a web portion 54 that is sized to threadingly mate with a secondary bushing 56 that in turn threadingly mates with an eye lens holder 58. The eye lens holder 58 includes an eye lens 60, and the bushing 56 supporting the eye lens holder is sealed to the primary hushing 28 by an elastomeric O-ring 62.

A washer 64 surrounds a nipple 66 inserted into the proximal end of the lens tube 32. A funnel-shaped member 68 is mounted inside the lens holder 58 opposite the lens 60. A spring 70, preferably of beryllium copper, biases between the lens holder 58 and the wahser 64. In that manner, the focus of the optic lens 60 is adjustable by threadingly adjusting the position of the lens holder 58 with respect to the secondary sleeve 58 against the bias of spring 70.

The eye piece 18 is of a polymeric material, preferably an acetal compound such as DELRIN. The eye piece 18 is provided with a cylindrically-shaped portion 72 that fans out into a delta portion 74 having an internal opening 76. The cylindrically-shaped eye piece portion 72 is threadingly mated to the housing sleeve 30 and sealed therein by an O-ring 78. A pane 80 of optical glass is mounted in an internal opening 76 to protect the interior of the eye piece 18 and the lens holder 58. In use, a person places their eye against the pane 80 of the eye piece 18 and looks at the lens 60 to view an object of interest proximate the distal end of the lens/light conduit 14.

As shown in Fig 2C, the distal end of the lens tube 32 terminates at an acute angle with respect to the longitudinal axis of the lens/light conduit 14. A wide angle lens 82 (Figs. 2C and 3) is located at the distal end of the lens/light conduit 14 to capture a broad area of reflective images. These images are transmitted to a first reflective mirror 84 and then a second reflective mirror 86 that directs the reflected light to a first rod lens system 88 consisting of a first convex lens 90 and a first concave lens 92 separated by a brass spacer 94. The first rod lens system 88 reflects an inverted image to a second rod lens system 96 which inverts the image 180° to a third rod lens system 98 that inverts the image 180° to a fourth rod lens system 100 which in turn inverts the reflected image 180° to a fifth rod lens system 102 and finally to the optic lens 60 located in the eye lens holder 58. The second, third, fourth and fifth lens systems 96, 98, 100 and 102, respectively, are similar to the first lens system 88, and the spacers thereof are provided to maintain proper spacing and alignment of the various convex and concave lenses. The lens can be Hopkins rod-type lens.

Thus, the present invention has been shown and described with respect to an exemplary endoscope having its metal parts made of brass material specifically formulated for use in close proximity to an MRI scanner. The brass material provides a 1:1 image artifact, exhibits substantially no attraction to the scanner under the deflection angle test, which is a standard well known in the art, and provides a specific absorption rate well within acceptable ranges.

In accordance with the stated low magnetic susceptibility characteristics of the endoscope 10 of the present invention, Table 1 lists the magnetic susceptibilities of the various materials used to construct the endoscope along with selected other materials.

**TABLE 1**

| Material | Density (g/cc) | Atomic or Molecular Weight | Susceptibility (x 10⁶) |
|---|---|---|---|
| Carbon (polycrystalline graphite) | 2.26 | 12.011 | -218 |
| Gold | 19.32 | 196.97 | -34 |
| Beryllium | 1.85 | 9.012 | -24 |
| Silver | 10.50 | 107.87 | -24 |
| Carbon (diamond) | 3.513 | 12.011 | -21.8 |
| Zinc | 7.13 | 65.39 | -15.7 |
| Copper | 8.92 | 63.546 | -9.63 |
| Water (37°C) | 1.00 | 18.015 | -9.03 |
| Human Soft Tissues | ∼1.00-1.05 | - | ∼(-11.0 to - 7.0) |
| Air (NTP) | 0.00129 | 28.97 | +0.36 |
| Stainless Steel (non-magnetic, austenitic) | 8.0 | - | 3520-6700 |
| Chrome | 7.19 | 51.996 | 320 |

In contrast, Table 2 lists the magnetic susceptibilities of various relatively highly magnetic materials.

**TABLE 2**

| Material | Density (g/cc) | Atomic or Molecular Weight | Susceptibility |
|---|---|---|---|
| Nickel | 8.9 | 58.69 | 600 |
| Stainless Steel (magnetic, martensitic) | 7.8 | - | 400-1100 |
| Iron | 7.874 | 55.847 | 200,000 |

The data used to construct Tables 1 and 2 was obtained from a paper authored by John Schneck of General Electric Corporate Research and Development Center, Schenectady, New York 12309, entitled "The Role of Magnetic Susceptibility In Magnetic Resonance Imaging: Magnetic Field Compatibility of the First and Second Kinds". The disclosure of that paper is incorporated herein by reference.

The following examples describe the manner and process of manufacturing a medical device according to the present invention, and they set forth the best mode contemplated by the inventors of carrying out the invention, but they are not to be construed as limiting.

### EXAMPLE I

Six (6) brass tubes supplied by Comeg Endoscopy, Tuttlinger, Germany having different inside diameters and wall thicknesses were tested for artifact under MR imaging. Dimensions of the tubes are listed in Table 3. Length and mass measurements were determined using vernier calipers (C-069) and a Mettler balance (Model PR803, ID #450282).

**TABLE 3**

| Brass Tube | Outside Diameter (mm) | Inside Diameter (mm) | Length (mm) | Mass (g) |
|---|---|---|---|---|
| 1 | 4.0 | 3.2 | 152.2 | 5.689 |
| 2 | 5.6 | 4.5 | 152.5 | 10.114 |
| 3 | 5.6 | 5.0 | 152.3 | 6.233 |
| 4 | 6.0 | 5.5 | 152.3 | 5.835 |
| 5 | 8.0 | 7.2 | 152.3 | 5.769 |
| 6 | 8.0 | 7.4 | 152.2 | 8.920 |

Each brass tube was tested in a General Electric (GE) 64 MHz 1.5 Tesla MR system at the location of the maximum spatial gradient of the field. Artifact assessment was carried out with the brass tubes inserted into a turkey breast phantom to simulate tissue interaction with the tubes. The send/receive head coil (for improved signal to noise) was used in conjunction with the following pulse sequence to assess artifact size:
fast spoiled gradient recalled echo in the steady state (FSPGR)
repetition time: 50 msec
echo time: 4 msec
flip angle: 30°
matrix size: 256 x 128
section thickness: 3 mm
field of view: 14 cm
number of excitations: four
imaging plane: axial

Artifact size was rated in accordance with the established system used by Dr. Frank Shellock at R&D Services, Inc., Los Angeles, Calif. and described in the following publications, which are incorporated herein by reference:
(1) Shellock FG, Crues JV: High-field MR imaging of metallic implants: an ex vivo evaluation of deflection forces. American Journal of Roentgenology. 151:389-392, 1988.
(2) Shellock FG, Schatz CJ: High field strength MRI and otologic implants. American Journal of Neuroradiology. 12:279-281, 1991.
(3) Shellock FG, Meeks T: Ex vivo evaluation of ferromagnetism and artifacts for implantable vascular access ports exposed to a 1.5 T MR scanner. Journal of Magnetic Resonance Imaging. 1:243, 1991.
(4) Shellock FG, Morisoli SM. Ex vivo evaluation of ferromagnetism, heating, and artifacts for heart valve prostheses exposed to a 1.5 Tesla MR system. Journal of Magnetic Resonance Imaging. 4:756-758, 1994.

The FSPGR is a partial flip angle technique that tends to have the greatest degree of artifact associated with it when MR imaging is performed on a metallic implant and, therefore, represents a "worst case" condition.

Artifact "size" was graded according to the following scheme: (neg) equals no artifact; (+) indicates an artifact less than the size of the device; (++) indicates an artifact the same as the size of the device; (+++) indicates an artifact slightly larger than the size of the device; and (++++) indicates an artifact larger than twice the size of the device.

The artifact test results are set forth in Table 4.

**TABLE 4**

| BRASS TUBE | ARTIFACT |
|---|---|
| 1 | ++ |
| 2 | ++ |
| 3 | ++ |
| 4 | ++ |
| 5 | ++ |
| 6 | ++ |

According to the artifact test results, the extent of the artifact displayed a 1:1 ratio, which is extremely acceptable from an MR imaging standpoint.

### EXAMPLE II

The six brass tubes used in Example I were subjected to deflection testing at R&D Services, Inc. All tests performed to determine magnetic field attraction were conducted at the position in the 1.5 MR system where the spatial gradient of the field is at a maximum, according to the methods reported in the above-referenced publications by Dr. Shellock. Such ex vivo testing of ferromagnetism involved the use of standardized procedures, indicated as the "deflection angle" test.

The results of the tests indicated that none of the brass samples exhibited any magnetic field attraction during exposure to the 1.5 Tesla static magnetic field of the MR system.

### EXAMPLE III

An exemplary one of the brass tubes (5.6 mm OD x 4.5 mm ID x 122.6 mm long, mass of 9.810g) used in Examples I and II was submitted to Dirats Laboratories, Westfield, Mass., for chemical analysis. The nominal composition of the brass tube and results from the chemical analysis are summarized in Table 5 (weight percent).

**TABLE 5**

| Cu | Zn | Pb | Sn | Fe | Si | Cd | O |
|---|---|---|---|---|---|---|---|
| 64.36 | 35.6 | 0.002 | <0.002 | 0.004 | <0.002 | <0.002 | 0.0034 |

The chemical composition of the brass material supplied by Comeg Endoscopy was shown to be predominantly copper and zinc with a minimal amount of iron. Although iron is present in the brass material, the amount indicated from the analysis does not cause attraction in the presence of a 1.5 Tesla static magnetic field, nor does it negatively affect artifact generation during MR imaging. These tests demonstrate that the brass tube material of the present invention is acceptable for use in MR interventional surgical tools.

### EXAMPLE IV

During magnetic resonance procedures, a large portion of the radio frequency (RF) power transmitted to the patient is transformed into heat within the patient's tissue. Surgical tools used in the magnetic resonance (MR) system also experience RF heating. Localized burning as well as internal biological changes can occur within the tissue of a patient and thus pose a risk. Therefore, materials that experience minimum temperature increases from RF heating are desirable for use within an MR system. This example compares the RF heating properties of both a brass tube and a machined, tapered brass rod.

Two liters of a phantom solution, 1 g CuSO₄ per liter of deionized water were poured into a plastic container of dimensions 44.5 mm x 171.5 mm x 323.9 mm. A plastic rack was lowered into the phantom solution and fixed in place using adhesive tape. The rack served to support the samples at a fixed height relative to the base of the container.

The plastic container with the phantom solution was placed on the sliding table of the MR system and allowed to equilibrate with the magnetic resonance system room temperature for three hours.

Type-K thermocouple wire (chromel/alumel, accuracy ±2°C) was provided in a length of 9.14 meters to allow temperature measurements, as indicated by a Keithley Digital Thermometer (Model 871, serial number 232097), outside of the high strength magnetic field of the MR room. The thermocouple wire was highly attracted to the magnet of the MR system and was affixed to the sliding table with adhesive tape. The thermocouple wire was also taped to the floor in the MR room, taped along the door sill, and taped to the floor outside of the MR room. Care was taken to prevent the thermocouple wire from coiling or looping which, during imaging, could induce electrical currents in the presence of the high magnetic field strength of the MR system.

The first sample was a brass tube having a mass of 1.902g. The brass tube was 3 inches in length x .125 inches OD x .097 inches ID. The second sample, a solid brass rod, was machined to the dimensions of 6 inches long with an OD of .125 inches for five inches then tapering for the last inch to a point having an OD of .04 inches. The machined brass rod had a mass of 9.206g.

The thermocouple was attached to one end of the brass tube with adhesive tape and the sample was immersed in the phantom solution, supported by the plastic rack. The sliding table was retracted into the magnet so that the plastic container was positioned in the body coil of the MR system. The ventilation fan was turned off and the MR room door closed. The digital thermometer was turned on and a baseline temperature of the phantom solution/sample was recorded.

Testing was conducted using the same GE Signa 1.5T magnetic resonance system used in the previous Examples I and II. Each brass sample was MR tested individually. MR parameters selected were previously used by Dr. Frank Shellock in determining RF heating on surgical instruments and are as follows:
T1 weighted spin echo pulse sequence
total imaging time: 26 minutes
axial plane: 134 msec
echo time: 25 msec
field of view: 48 cm
imaging matrix: 256 x 128
section thickness: 20.0 mm
number of section locations: 1
number of excitations: 94
number of echos: 4
phasing direction: anterior to posterior
transmitter gain: 200

Temperature measurements were recorded at one minute intervals, as indicated by the MR system timer.

The brass tube attained a maximum temperature of 21.1°C during the 26 minute MR scan (mass of 1.902 g). Once the MR scan was terminated, the temperature immediately dropped to 20.3°C, 0.3°C above the initial baseline temperature of 20.0°C. The maximum change in temperature was 1.1°C during the scan.

The solid brass rod attained a maximum temperature of 20.9°C during the 26 minute MR scan (mass of 9.206 g). Once the MR scan was terminated, the temperature immediately dropped to 20.6°C, 0.2°C above the initial baseline temperature of 20.4°C. The maximum change in temperature was 0.5°C during the scan.

The calculated whole body averaged specific absorption rate (SAR) was 0.122 W/kg for both the brass tube and the solid brass rod using a patient weight of 90 lbs. This absorption rate is acceptable for the present medical devices used in interventional MR assisted procedures.

Thus, the endoscope of the present invention is an instrument which is useful for pre- and post-clinical and surgical applications, especially in an environment proximate the strong magnetic field emitted by a magnetic resonance imaging scanner.

It is appreciated that various modifications to the inventive concepts described herein may be apparent to those of ordinary skill in the art without departing from the spirit and scope of the present invention as defined by the appended claims.

## Claims

1. A device that is useful proximate the magnetic field emitted by a magnetic resonance imaging scanner, which comprise:
a body comprising at least some metal-containing parts of a brass material having, by weight percent, about 62 to 65% copper, remainder zinc.

2. The device of claim 1 wherein the brass material further comprises, by weight percent, less than about 0.02% cadmium, less than about 0.03% iron, less than about 0.03% lead and less than about 0.03% tin.

3. The device of claim 2 wherein the brass material further comprises, by weight percent, less than about 0.002% silicon and about 0.0034% oxygen.

4. The device of claim 1 as a hand held medical device.

5. The device of claim 1 wherein the brass material provides a 1:1 artifact under the influence of a GE 64 MHz 1.5 Tesla MR system at a location of maximum spatial gradient of the field.

6. The device of claim 1 wherein the brass material exhibits substantially no attraction in the present of a GE 64 MHz 1.5 Tesla Mr system under the deflection angle test.

7. The device of claim 1 wherein under the influence of a magnetic field generated by a GE 64 MHz 1.5 Tesla MR system, the brass material provides a specific absorption rate of about 0.122 w/kg based on a patent weight of 90 lbs.

8. The device of claim 1 as an endoscope.

9. The device of claim 8 wherein the endoscope is selected from the group consisting of a sinuscope, an arthroscope, a laparoscope, a laryngoscope and a hysteroscope.

10. The device of claim 1 wherein the brass is plated with chrome.

11. The device of claim 10 wherein the brass is chrome plate without a nickel strike.

12. A method of providing a device that is useful proximate the magnetic field emitted by a magnetic resonance imaging scanner, comprising:
providing the device having a body comprising at least some metal-containing parts of a brass material having, by weight percent, about 62 to 65% copper, remainder zinc.

13. The method of claim 12 including providing the brass material further comprising, by weight percent, less than about 0.02% cadmium, less than about 0.03% iron, less than about 0.03% lead and less than about 0.03% tin.

14. The method of claim 13 including providing the brass material further comprising, by weight percent, less than about 0.002% silicon and about 0.0034% oxygen.

15. The method of claim 12 including providing this device as a hand held medical device.

16. The method of claim 12 including providing the brass material having a 1:1 artifact under the influence of a GE 64 MHz 1.5 Tesla MR system at a location of maximum spatial gradient of the field.

17. The method of claim 12 including providing the brass material exhibiting substantially no attraction in the presence of a GE 64 MHz 1.5 Tesla MR system under the deflection angle test.

18. The method of claim 12 including providing the device having a specific absorption rate of about 0.122 w/kg using a patient weight of 90 lbs. under the influence of a magnetic field generated by a GE 64 MHz 1.5 Tesla MR system.

19. The method of claim 12 providing the device as an endoscope.

20. The method of claim 12 including directly plating a chrome coating on the brass.
